# EUROPEAN PATENT SPECIFICATION

(11) **EP 4 079 798 B1**
(45) Date of publication and mention of the grant of the patent: **16.07.2025**
(21) Application number: 20902056.9
(22) Date of filing: 20.11.2020
(51) Int. Cl.: C08J 11/06, B32B 15/20, B32B 1/00, B29B 17/02, C07C 69/82, C08J 11/08

(54) **PROCESS FOR RECYCLING LAMINATED POLYMER PACKAGING USING ETHYLENE GLYCOL**
VERFAHREN ZUR WIEDERVERWERTUNG VON LAMINIERTEN POLYMERVERPACKUNGEN UNTER VERWENDUNG VON ETHYLENGLYKOL
PROCÉDÉ DE RECYCLAGE D'EMBALLAGES POLYMÈRES STRATIFIÉS AU MOYEN D'ÉTHYLÈNE GLYCOL

(30) Priority: 19.12.2019 BR 102019027314
(43) Date of publication of application: 26.10.2022
(73) Proprietor: Joaquim Antunes Quevedo, Edson, 06465-136 Barueri (BR); Silva de Araujo, Henrique, 13272-554 Valinhos (BR)
(72) Inventor: Joaquim Antunes Quevedo, Edson, 06465-136 Barueri (BR); Silva de Araujo, Henrique, 13272-554 Valinhos (BR)
(74) Representative: ZBM Patents - Zea, Barlocci & Markvardsen
(86) International application number: PCT/BR2020/050494
(87) International publication number: WO 2021/119776

(56) References cited:
- EP-A1- 1 227 075
- EP-A1- 1 437 377
- EP-A1- 1 437 377
- EP-B1- 1 683 829
- EP-B1- 1 683 829
- WO-A1-2014/098229
- CN-A- 109 134 921
- DE-A1- 19 744 436
- US-A1- 2017 008 826

## Description

### Field of Application

The present invention refers to the field of application of industrial chemical processes, more precisely in the recycling processes of polymeric packaging with laminated structure 03/06.

The recycling process of the present invention is intended for the recycling of polymeric packaging with a laminated structure and stands out from other recycling processes, as it allows the separation of polymers joined by lamination, which are incompatible with the conventional thermoplastic reprocessing process.

Specifically, the technique achieves the complete separation of PET (polyethylene terephthalate) through its selective dissolution in the medium and the separation of PE (polyethylene) and PP (polypropylene) through its complete melting, in a system of heterogeneous phases, wherein they can be collected directly, thus allowing the recycling of any polymeric packaging independent of its additives. Said packaging can be manufactured in laminated films containing PET in its metallic form or not, aluminium, polyethylene and polypropylene in its metallic form or not. In order to perform the separation, a process comprising ethylene glycol in its mono-, di- or tri-forms is applied, allowing the dissolution of the packaging's polyester films, resulting in elements that can return to the manufacturing process of new packaging, configuring a circular economy.

### Description of Prior Art

Currently, one of the most commented and widespread subjects, both in academia and in industry, concerns the care for the environment, as well as the rational use of natural resources. Thus, concepts such as sustainability, circular economy and recycling are always in the spotlight and in a position to improve.

It is not new that, with the process of global industrialization, there has been an intensification of natural resources use and, consequently, environmental degradation has also escalated, causing the debate on the best use of natural resources and, thus, the reduction of damages caused to the environment, became increasingly urgent.

From this need, much progress has been made regarding the reuse of natural resources and the efficiency of recycling processes of the most diverse products of daily use.

However, some products stand out negatively since their recycling process is difficult, such as multilayer laminated packaging. Such packaging does not have effective recycling processes, represent a large part of the domestic waste generated in cities and are also the main pollutants of the oceans.

Undoubtedly, this type of packaging has remarkably interesting characteristics and, consequently, great industrial application, whether as sealing films, food storage and in the packaging of industrial raw materials, among others, so that its use becomes almost essential.

Recycling of this type of laminated packaging is not effective since they are made up of several elements such as, for example, layers of laminated polyethylene terephthalate, both in its metallic form and in its normal form, aluminium, polyethylene, polypropylene in both its metallic form and in its normal form, in addition to any impressions that the packaging may have. Within this variety of packaging elements, the most critical elements are PET and aluminium, since their physical characteristics are quite different in terms of processing and extrusion, when compared to other polymers such as PE or PP, so that the combination of PET and/or aluminium in packaging makes it impossible to reuse PE or PP and vice versa.

Facing this situation, the prior art shows alternatives that try to make the recycling processes of polymeric packaging with laminar structure viable, such as, for example, PI0706115-3, filed on 10/22/2007 intitled "RECICLAGEM DE EMBALAGEM MULTICAMADAS", owned by UNICAMP. According to its abstract, the patent document refers to a process of separating the materials making up a multilayers packaging. More specifically, said process allows said multilayers making up plastic, metallic and paper materials to be separated so as not to generate residues harmful to the environment.

Document PI0706115-3, filed on 10/22/2007 intitled "RECICLAGEM DE EMBALAGEM MULTICAMADAS", shows two steps, the first of which is an immersion bath in an NaOH alkaline medium in order to separate the plastic layer the packaging of the metallic layer; and the second step consisting of a new immersion bath in a NaOH-based alkaline medium, reacting directly with the metallization, resulting in Al³⁺, which is subsequently treated.

The disadvantage of the process showed by document PI0706115-3 lies in the fact the process has only a solution for the metallic layer of the laminated structure packaging. Therefore, polymeric compounds require further processing, making the process taught by the document ineffective, in addition to not acting in the wide universe of packaging that combines PET and PE or PP without having at the joining interface a metallic layer or aluminium film.

Document BR102013023494-0, filed on 09/13/2013 intitled "PROCESSO DE SEPARAÇÃO E RECICLAGEM QUÍMICA DE EMBALAGENS MULTICAMADAS", shows, according to its abstract, an alternative for the recycling of multilayer packaging containing PET (Polyethylene terephthalate), aluminium and PE (Polyethylene) by chemical recycling of this type of packaging through the delamination of polymers and the depolymerization of PET using hydrolysis reaction. A further method for recycling laminate polymeric packaging comprising PET, PE, PP and aluminium involving selective dissolution is known from document EP 1 683 829 B1.

Document BR102013023494-0 introduces the use of an alcohol for recycling the laminated packaging, however, the product of the process will still result in PE and PET together so that the extrusion of the polymers will be unfeasible given the difference in density of both.

Some other solutions are showed in other patent documents such as US2009011213 and WO2018025058 which share the same difficulties, that is, they have processes based on a NaOH alkaline bath, which attacks the aluminium metallization layer through selective dissolution, however do not allow the reuse of polymers such as PE and PP, or PET, whenever there is PET directly bonded to PE or PP without aluminium interleaved in the bonding interface.

Therefore, the prior art would benefit from a process that allows the complete separation of PET through its selective dissolution in ethylene glycol, as well as the separation of PE and PP films through their complete melting in the medium, generating a system of heterogeneous phases, in which PP and PE can be collected directly on the medium surface and wherein aluminium, if present, can be collected in its metallic form through the formation of a precipitate (decantation) so that the PET and the other mentioned components can be separated and reused.

### Summary of Invention

The present invention aims to show a recycling process applied to polymeric packaging with a laminated structure, which allows the separation and reuse in a circular economy of its respective layers, regardless of the way or sequence in which they are combined.

Another objective of the invention is to show a process allowing the aluminium layer to be reused in its metallic form, and not as salts.

It is also an objective of the present invention to show a process wherein the bath is used as a raw material in the production of PET, thus configuring a circular economy.

### Brief Description of the Invention

The invention achieves the described objectives from the use of an ethylene glycol-based bath, being able to use its forms: mono-, di- or tri- instead of an NaOH alkaline bath.

Considering that ethylene glycol is a manufacture precursor of polyethylene terephthalate (PET), the product of the selective dissolution of PET in mono-ethylene glycol can be directly reintroduced in the polycondensation step in the PET manufacture, serving as raw material for this process.

PET final form after bathing allows its reuse through chemical recycling into the production of new PET polymers, thus configuring a circular economy with minimal material loss.

The ethylene glycol bath also allows an effective separation of the aluminium layer in a reaction resulting in metallic aluminium, which has greater economic value compared to aluminium salts obtained in a NaOH bath.

Finally, the ethylene glycol reaction itself results in a compound that can return to PET production, requiring only enrichment with terephthalic acid in the polycondensation step, so that the reuse of materials is almost complete.

### Brief Description of the Drawings

The subject matter of this Invention will be fully clear in its technical aspects from the detailed description that will be made based on the figures listed below, wherein:

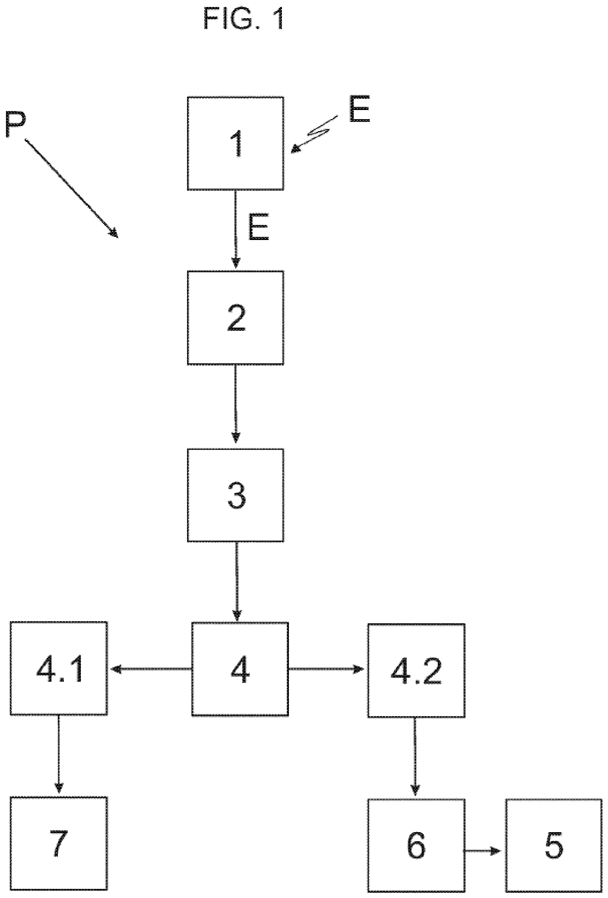

Figure 1 shows a block diagram referring to the steps and sub-steps comprised by the polymeric packaging recycling process through ethylene glycol, referring to the correlation between them.

### Detailed Description of the Invention

In accordance with the objectives showed through the brief description, the present patent application "PROCESS FOR RECYCLING LAMINATED POLYMERIC PACKAGING USING ETHYLENE GLYCOL" shows a process (P) aiming at the recycling of polymeric packaging (E) endowed with a laminar structure comprising PET, PE, PP and/or aluminium, regardless the amount or combination between them.

In this process (P), PET is separated through a chemical reaction, while aluminium, PE and PP are separated through physical processes. In detail, PET is chemically separated through its complete selective dissolution, which takes place through a reaction with glycol, which can be treated as: glycolysis, transesterification or alcoholysis in the literature. PE and PP are physically separated when they reach a temperature above their melting points, then undergo melting and, as they form a heterogeneous system with glycol, float in the solution. Aluminium remains solid, without the presence of any adhesive polymer. Finally, the metallic aluminium precipitates and settles to the bottom of the bath.

The process (P) has the following consecutive steps:
(i) granulation (1) of the polymeric packaging (E);
(ii) immersion (2) of the polymeric packaging (E) in an ethylene glycol bath;
(iii) selective dissolution (3) of the polymeric packaging (E);
(iv) separation (4) of polymers;
(v) washing (5) the polymers;
(vi) griding (6) the polymers;
(vii) separation of aluminium (7) after the selective dissolution step (3); and
(viii) cleaning and drying (8) of the aluminium.

The granulation step (1) of the polymeric packaging (E) consists of fragmenting the packaging into small portions, aiming at the smallest possible particle size, in order to accelerate the PET selective dissolution step (3).

The granulation step (1) is performed with the aid of industrial equipment capable of cutting and grinding the packaging (E). Said industrial equipment also performs the cleaning of said packaging (E) by passing them after cutting and grinding into a gutter with water. After the packaging (E) has passed through the gutter with water, they are dried by the industrial equipment itself through a centrifuge and air blowing process.

The immersion step (2) takes place after the granulation step (1), so that the packaging (E) is cut. This immersion step (2) is performed in a chemical reactor, preferably made of stainless steel; and in an inert atmosphere with N₂, wherein the aforementioned chemical reactor has an upper inlet for nitrogen purge at a flow between five to ten L/min.

Still in the immersion step (2), the packaging (E) is immersed in a pure glycol solution, preferably in mono-ethylene glycol, which can be performed in di-ethylene glycol or tri-ethylene glycol, however, not being limited to these types of glycols. Pure glycol comprises molecular structure I shown below: 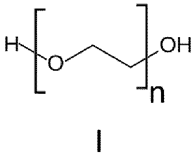 wherein, brackets "[ ]" represent a single glycol unit and "n" is the index referring to the number of glycol units for a given pure glycol used in the process (P). For the purposes of the present invention, the value of "n" comprises the range between 1 and 10, preferably n = 1, n = 2 or n = 3, however, not being limited to these values. When n = 1, n = 2 and n = 3 the resulting pure glycol is, respectively, mono-ethylene glycol, di-ethylene glycol and tri-ethylene glycol.

The immersion step (2) may comprise heating and stirring sub-steps, so that in the heating sub-step the glycol is heated by means of a thermal fluid heater, heating the reactor via a coil, raising the temperature of the glycol to a range between 180 °C and 240 °C, with respect to the glycol boiling temperature used, that is, 197.6 °C for mono-ethylene glycol, 245 °C for di-ethylene glycol and 285 °C for tri-ethylene glycol. A top condenser can be installed to recover steams from the glycol, increasing process throughput.

At stirring sub-step of the immersion stage (2), the chemical reactor is stirred through a system comprising a motor and a geared motor, so that the packaging (E) is uniformly mixed with the glycol, thus facilitating the PET selective dissolution. As the immersion step (2) is performed in a closed vessel condition, the volume of packaging (E) must respect the range between 40 to 60% of the glycol volume, thus facilitating the separation of the supernatants PE and PP and precipitated aluminium.

The selective dissolution step (3) of the packaging (E) starts after the stabilization of the glycol temperature at the immersion step (2). Said selective dissolution step (3) can be accelerated, that is, with the glycol at the appropriate temperature, the immersed packaging (E) is accelerated by means of Cowles-type propellers, circulation and/or pumping gears or circulation through screens metallic, so that acceleration contributes to the shearing of the supernatant material composed of PE or PP that can trap particles to be separated.

The selective dissolution step (3) time will depend on the size of the packaging (E), more precisely on the size of the portions achieved in the granulation step (1), ranging from 20 to 60 minutes. A visual indicator of the selective dissolution step (3) end is that it will not be possible to visualize films suspended in the glycol, with only a kind of supernatant sludge corresponding to PP and PE being visible. At the selective dissolution step (3) end, the result of the glycol and the packaging (E) reaction will be a suspension of PP and PE, or one of the two, depending on the initial configuration of the packaging; aluminium precipitated in its metallic form, if the packaging (E) contains aluminium; and at least one type of terephthalic acid-based polyol.

The chemical reaction between glycol and PET, called glycolysis, transesterification or alcoholysis, is represented below for the purpose of better understanding of the present invention, however, not being limited to this type of reaction. In this non-restrictive example, the glycol, of molecular structure I, chemically reacts with PET, of molecular structure **II,** to form a first type of terephthalic acid-based polyol of molecular structure III, being a by-product of the reaction. In this reaction, regardless of the glycol type used for the reaction with PET, another by-product formed will be mono-ethylene glycol, of molecular structure IV, as this by-product comes from the mono-ethylene units present in PET. The molecular structure II of PET comprises "m" terephthalate units represented in brackets "[ ]," so that "m" is equal to or greater than 1. Said molecular structure III polyol comprises "x" terephthalate units represented in brackets "[ ]," so that "x" is equal to or greater than 1 and has values lower than "m". In this context, "m" will always be greater than "x" (m > x). Still on said polyol of molecular structure III, it can indefinitely undergo further reactions with the mono-ethylene glycol by-product of molecular structure IV or with the glycol of molecular structure I, in case of excess of said glycol in the medium, resulting in a second type of terephthalic acid-based polyol of molecular structure V, a product of the complete reaction. This second type of polyol comprises the bis(2-hydroxyalkyl) terephthalate product, however, not being limited to that second type of polyol. Although complete reaction between PET and glycol is desired, at least two types of polyol can be simultaneously present. In a reaction between PET and glycol comprising values of n = 1 (mono-ethylene glycol), 2 (di-ethylene glycol), 3 (tri-ethylene glycol), 4 (tetra-ethylene glycol), 5 (penta-ethylene glycol), 6 (hexa-ethylene glycol), 7 (hepta-ethylene glycol), 8 (octa-ethylene glycol), 9 (nona-ethylene glycol) or 10 (deca-ethylene glycol), the alkyl function of the second type of polyol formed bis(2-hydroxyalkyl) will be selected, respectively, from the group comprising ethylene, di-ethylene, tri-ethylene, tetra-ethylene, penta-ethylene, hexa-ethylene, hepta-ethylene, octa-ethylene, nona-ethylene or deca-ethylene, however, not being limited to said values of "n" and the possible bis(2-hydroxyalkyl) products mentioned. Among numerous possibilities of the second type of polyol, the products bis(2-hydroxyethylene), bis(2-hydroxydi-ethylene) and bis(2-hydroxytri-ethylene) are preferred when the glycol comprises the values of n = 1, 2 or 3, respectively.

In the selective dissolution step (3), the glycol reacts in the packaging, specifically attacking the PET, so that the PET is dissolved, releasing the aluminium that precipitates in the glycol by density difference, while the PET reacts with the glycol forming at least one polyol. The PP and PE portions, on the other hand, melt due to the high temperature of the medium, but remain insoluble in the glycol, thus rising to the surface of the liquid, also due to the difference in density.

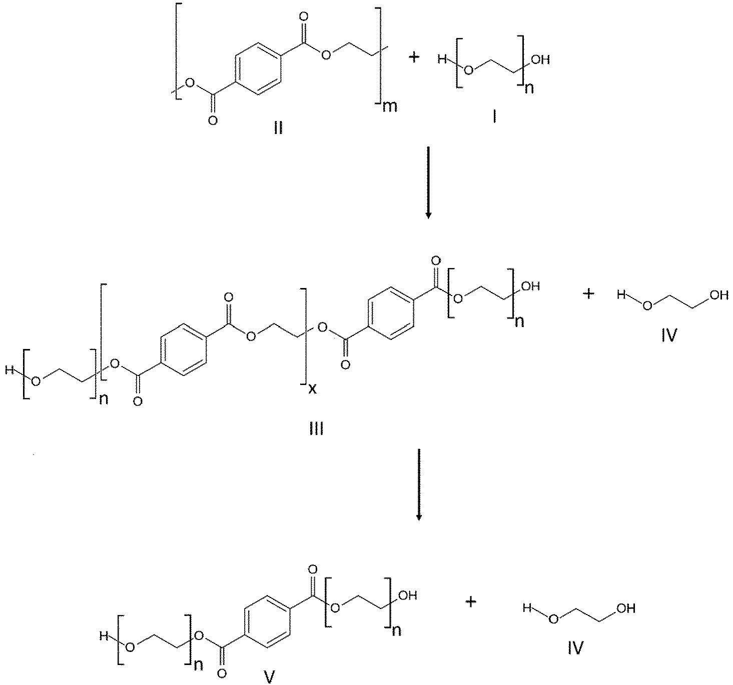

The separation step (4) occurs after the selective dissolution step (3) and complete melting of the PP and/or PE films. Said separation step (4) comprises the sub-steps of aluminium separation (4.1) and PE/PP separation (4.2).

The aluminium separation sub-step (4.1) consists of draining a small fraction of the bath from the bottom of the reactor, while hot, followed by passing the liquid from this bath through a filtration line, so that the aluminium that has already precipitated is retained and the glycol returned to the reactor. In order to facilitate the aluminium separation step (4.1), the reactor preferably has a conical or torispherical bottom, thus helping to remove the aluminium precipitated by the reactor bottom valve. In turn, the filtration line has a display through which it is possible to visualize the amount of aluminium retained, since the aluminium separation step (4.1) will only be completed when all the precipitated aluminium is retained in the filter line.

The PE/PP separation sub-step (4.2) consists of draining the bath and the supernatant portion through pumping, by means of side drains located in the reactor, directing them to a fine metallic screen filter, in order to remove or retain any unwanted particles which are adhered to the supernatant portion. After removing unwanted particles, the mixture between bath and supernatant portion is cooled so that the supernatant portion solidifies and is then physically removed, while the liquid from the bath is pumped back to the reactor.

The washing step (5) of the polymers takes place after the separation step (4) and, is performed through water to remove the glycol residues present both in PE and PP in an equipment similar to that used in the step of granulation (1). Thus, polymers are subjected to a grinding step (6), before the washing step (5) and then they go to a gutter with water, configuring the washing step (5), where all glycol residues are removed and, after decontamination, the polymers already cleaned are dried. Given the low solubility of glycol in PE or PP, the washing step (5) is highly effective, completely minimizing glycol particles.

The polymer grinding step (6) is performed before the washing step (5) in order to increase the contact area of the material with the water in the washing step (5).

The aluminium cleaning and drying step (7) takes place after the aluminium separation sub-step (4.1) and has the purpose of removing the glycol particles in it, preparing the material for later use. Said cleaning and drying step (7) of the aluminium is performed through water curtain-type direct rinsing on a conveyor or into a gutter.

The polyols resulting from the selective dissolution of

PET can be reused in two ways, the first being as a reagent in consecutive processes, returning to the reactor as a bath. However, the use of polyols in consecutive processes gradually increases its viscosity, due to the reaction between PET and the polyols itself, so that their consecutive use will only be possible as long as their viscosity allows.

The second way is when the glycol already has a viscosity that prevents its use in consecutive processes, being subjected to a re-polymerization process through the addition of terephthalic acid in the PET polycondensation. To know the amount of terephthalic acid needed for the re-polymerization process, it is essential to measure the number of free hydroxyl of the polyols obtained (mgKOH/g).

The great advantage of the process (P) compared to other polymeric packaging (E) recycling processes with a laminated structure is to show an economically viable and effective way to reuse the PET present in this type of polymeric packaging (E). Not to mention that this process manages to completely remove PET from other materials, and this is a great advantage, since the presence of PET waste creates important technical problems in the recovery of PE and PP, as it has quite different melting characteristics and this is the main practical reason recycling of multi-layer laminates is currently unfeasible.

Another particularly important advantage of the aforementioned process (P) lies in the fact that it configures a circular economy, that is, all products from the process (P) can be re-used in the polymeric packaging (E) production chains themselves.

It should be understood the present description does not limit application to the details described herein and that the invention is capable of other embodiments and being practiced or performed in a variety of ways, within the scope of the claims. Although specific terms have been used, such terms should be interpreted in a generic and descriptive sense and not for the purpose of limitation.

## Claims

1. **A process for recycling laminated polymeric packaging using ethylene glycol** having a recycling process for polymeric packaging, wherein the process (P) is applied to polymeric packaging (E) comprising PET, PE, PP and aluminium, with the consecutive steps:
(i) granulation (1) of the polymeric packaging (E);
(ii) immersion (2) of the polymeric packaging (E) in an ethylene glycol bath;
(iii) selective dissolution (3) of the polymeric packaging (E);
(iv) separation (4) of polymers, comprising the sub-steps of aluminium separation (4.1) and PE/PP separation (4.2);
(v) washing (5) of the polymers after the separation step (4), removing the glycol residues present in both PE and PP;
(vi) grinding (6) the polymers before the washing step (5);
(vii) cleaning and drying (7) the aluminium after the aluminium separation sub-step (4.1);
the washing (5) and grinding (6) steps are performed in an industrial equipment capable of cutting and grinding the packaging (E), having gutters with water and a centrifuge and air blowing drying system.

2. **The process,** according to claim 1, ***characterized in that*** the glycol comprises molecular structure I and PET comprises molecular structure II: 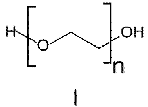 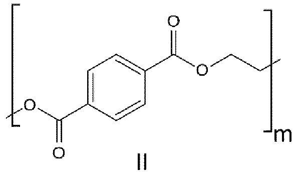 wherein:
- "n" is equal to values comprised in the range between 1 and 10, preferably between 1 and 3; and
- "m" is equal to or greater than 1.

3. The **process,** according to claim 1, ***characterized in that*** the granulation step (1) fragments the polymeric packaging (E) into small portions in an industrial equipment capable of cutting and grinding the packaging (E), having gutters with water and centrifuge and air blow drying system.

4. The **process,** according to claims 1 and 3, ***characterized in that*** the immersion step (2) is performed after the granulation step (1) in a chemical reactor preferably built of stainless steel and in a controlled atmosphere with N₂, and the chemical reactor having an upper inlet for nitrogen purge at a flow between five to ten L/min; and a conical or torispherical bottom.

5. The **process,** according to claim 4, ***characterized in that*** the immersion step (2) is performed in a pure glycol bath, in a closed vessel condition, and the volume of packaging (E) must respect the range between 40 to 60% glycol volume.

6. The **process,** according to claim 4, ***characterized in that*** the immersion step (2) may comprise heating and stirring sub-steps, wherein the stirring sub-step takes place through a system comprising motor and geared motor.

7. The **process,** according to claim 6, ***characterized in that*** the heating sub-step is performed through a thermal fluid heater containing coil, raising the glycol temperature to a range between 180 °C and 240 °C.

8. The **process,** according to claims 1 and 6, ***characterized in that* the** selective dissolution step (3) is performed after the glycol temperature stabilization in the immersion step (2) lasting between twenty and sixty minutes.

9. The **process,** according to claim 8, ***characterized in that*** the selective dissolution step (3) is accelerated through Cowles-type propellers, circulation and/or pumping of gears or circulation through metallic screens.

10. The **process,** according to claim 8, ***characterized in that*** the product of the selective dissolution step (3) will be a suspension of PP and PE; aluminium precipitated in its metallic form; and at least one terephthalic acid-based polyol comprising molecular structure III or molecular structure V: 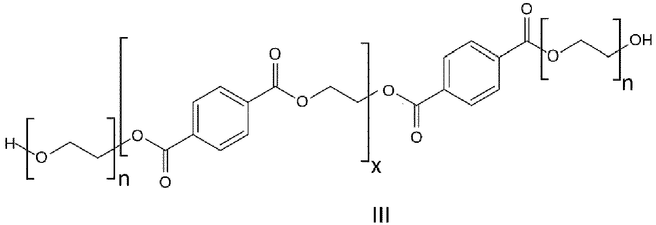 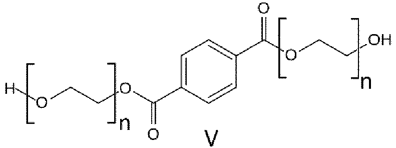 wherein:
- "n" is equal to values comprised in range between 1 and 10, preferably between 1 and 3;
- "x" is equal or greater than 1;

11. The **process,** according to claims 1 and 8, ***characterized in that*** the separation step (4) takes place after the selective dissolution step (3) and complete melting of the PP and/or PE films, and the separation step (4) comprising the aluminium separation (4.1) and PE/PP separation (4.2) sub-steps.

12. The **process,** according to claim 11, ***characterized in that*** the aluminium separation sub-step (4.1) consists of draining a small fraction of the bath from the reactor bottom, under heat, followed by passing this bath through a filtration line.

13. The **process,** according to claim 11, ***characterized in that*** the PP/PE separation sub-step (4.2) consists of draining the bath and the supernatant portion through pumping, through side drains located in the reactor, directing them to a fine metallic screen filter, cooling the supernatant portion and returning the bath to the reactor by pumping.

14. The **process,** according to claims 1 and 4, ***characterized in that*** the process (P) uses mono-ethylene glycol, di-ethylene glycol and tri-ethylene glycol as glycol.

15. The **process,** according to claims 1 and 14, ***characterized in that*** the aluminium cleaning and drying step (7) takes place after the aluminium separation sub-step (4.1), removing the glycol particles at the aluminium through water curtain-type direct washing, on a conveyor or gutter.

## Patentansprüche

1. **Ein Verfahren zum Recycling von laminierten Polymerverpackungen unter Verwendung von Ethylenglykol** mit einem Recyclingverfahren für Polymerverpackungen, wobei das Verfahren (P) auf Polymerverpackungen (E) angewendet wird, die PET, PE, PP und Aluminium umfassen, mit folgenden aufeinanderfolgenden Schritten:
(i) Granulieren (1) der Polymerverpackung (E);
(ii) Eintauchen (2) der Polymerverpackung (E) in ein Ethylenglykolbad;
(iii) selektives Auflösen (3) der Polymerverpackung (E);
(iv) Abtrennen (4) von Polymeren, umfassend die Teilschritte von Aluminiumabtrennung (4.1) und PE/PP-Abtrennung (4.2);
(v) Waschen (5) der Polymere nach dem Abtrennschritt (4), Entfernen der Glykolreste, die sowohl im PE als auch im PP vorhanden sind;
(vi) Schleifen (6) der Polymere vor dem Waschschritt (5);
(vii) Reinigen und Trocknen (7) des Aluminiums nach dem Teilschritt der Aluminiumabtrennung (4.1);
wobei die Schritte des Waschens (5) und des Schleifens (6) in einer industriellen Ausrüstung durchgeführt werden, die in der Lage ist, die Verpackung (E) zu schneiden und zu schleifen und welche Rinnen mit Wasser und ein Zentrifugen- und Luftgebläse-Trocknungssystem hat.

2. **Das Verfahren** nach Anspruch 1, ***dadurch gekennzeichnet, dass*** das Glykol die molekulare Struktur I und das PET die molekulare Struktur II umfasst: 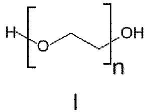 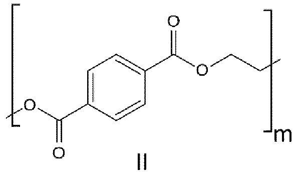 wobei:
- "n" gleich den Werten im Bereich zwischen 1 und 10, vorzugsweise zwischen 1 und 3 ist; und
- "m" gleich oder größer als 1 ist.

3. Das **Verfahren** nach Anspruch 1, ***dadurch gekennzeichnet, dass*** der Granulierungsschritt (1) die Polymerverpackung (E) in kleine Portionen in einer industriellen Ausrüstung fragmentiert, die in der Lage ist, die Verpackung (E) zu schneiden und zu schleifen, und welche Rinnen mit Wasser und ein Zentrifugen- und Luftgebläse-Trocknungssystem hat.

4. Das **Verfahren** nach den Ansprüchen 1 und 3, ***dadurch gekennzeichnet, dass*** der Eintauchschritt (2) nach dem Granulationsschritt (1) in einem chemischen Reaktor durchgeführt wird, der vorzugsweise aus rostfreiem Stahl gebaut ist, und in einer kontrollierten Atmosphäre mit N₂ und wobei der chemische Reaktor einen oberen Einlass für die Stickstoffspülung bei einem Durchfluss zwischen fünf und zehn L/min; und einen konischen oder torisphärischen Boden hat.

5. Das **Verfahren** nach Anspruch 4, ***dadurch gekennzeichnet, dass*** der Eintauchschritt (2) in einem Bad aus reinem Glykol in einem geschlossenen Gefäßzustand durchgeführt wird und das Volumen der Verpackung (E) den Bereich zwischen 40 und 60 % Glykolvolumen einhalten muss.

6. Das **Verfahren** nach Anspruch 4, ***dadurch gekennzeichnet, dass*** der Eintauchschritt (2) Teilschritte des Erwärmens und des Rührens umfassen kann, wobei der Teilschritt des Rührens mittels eines Systems erfolgt, das einen Motor und einen Getriebemotor umfasst.

7. Das **Verfahren** nach Anspruch 6, ***dadurch gekennzeichnet, dass*** der Teilschritt des Erwärmens durch eine thermische Fluidheizung, die eine Spule enthält, durchgeführt wird, wobei die Glykoltemperatur auf einen Bereich zwischen 180 °C und 240 °C erhöht wird.

8. Das **Verfahren** nach den Ansprüchen 1 und 6, ***dadurch gekennzeichnet, dass*** der Schritt des selektiven Auflösens (3) nach der Glykoltemperaturstabilisierung im Eintauchschritt (2) durchgeführt wird und zwischen zwanzig und sechzig Minuten dauert.

9. Das **Verfahren** nach Anspruch 8, ***dadurch gekennzeichnet, dass*** der Schritt des selektiven Auflösens (3) durch Propeller vom Cowles-Typ, Zirkulation und/oder Pumpen von Zahnrädern oder Zirkulation durch metallische Siebe beschleunigt wird.

10. Das **Verfahren** nach Anspruch 8, ***dadurch gekennzeichnet, dass*** das Produkt des Schritts des selektiven Auflösens (3) eine Suspension von PP und PE; in seiner metallischen Form ausgefälltes Aluminium; und mindestens ein Polyol auf Terephthalsäurebasis, das die Molekülstruktur III oder die Molekülstruktur V umfasst, sein wird: 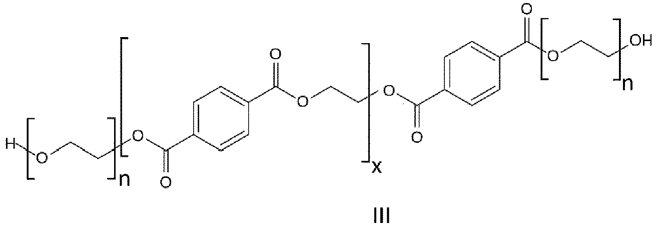 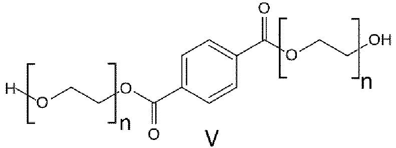 wobei:
- "n" gleich Werten im Bereich zwischen 1 und 10, vorzugsweise zwischen 1 und 3 ist;
- "x" gleich oder größer als 1 ist;

11. Das **Verfahren** nach den Ansprüchen 1 und 8, ***dadurch gekennzeichnet, dass*** der Abtrennschritt (4) nach dem Schritt des selektiven Auflösens und dem vollständigen Aufschmelzen der PP- und/oder PE-Folien stattfindet und der Abtrennschritt (4) die Teilschritte von Aluminiumabtrennung (4.1) und PE/PP-Abtrennung (4.2) umfasst.

12. Das **Verfahren** nach Anspruch 11, ***dadurch gekennzeichnet, dass*** der Teilschritt (4.1) der Aluminiumabtrennung darin besteht, einen kleinen Teil des Bades unter Wärme aus dem Reaktorboden abzulassen und dieses Bad dann durch eine Filtrationsleitung zu leiten.

13. Das **Verfahren** nach Anspruch 11, ***dadurch gekennzeichnet, dass*** der PP/PE-Abtrennungs-Teilschritt (4.2) darin besteht, das Bad und den überstehenden Teil durch Pumpen durch im Reaktor befindliche Seitenabläufe abzulassen, diese auf einen feinen metallischen Siebfilter zu leiten, den überstehenden Teil zu kühlen und das Bad durch Pumpen in den Reaktor zurückzuführen.

14. Das **Verfahren** nach den Ansprüchen 1 und 4, ***dadurch gekennzeichnet, dass*** das Verfahren (P) Mono-Ethylenglykol, Di-Ethylenglykol und Tri-Ethylenglykol als Glykol verwendet.

15. Das **Verfahren** nach den Ansprüchen 1 und 14, ***dadurch gekennzeichnet, dass*** der Aluminiumreinigungs- und -trocknungsschritt (7) nach dem Aluminiumabtrennungsunterschritt (4.1) stattfindet, wobei die Glykolpartikel am Aluminium durch eine Direktwäsche nach Art und Weise eines Wasservorhangs auf einem Förderer oder einer Rinne entfernt werden.

## Revendications

1. **Un procédé de recyclage d'emballages polymères stratifiés utilisant de l'éthylène glycol** ayant un procédé de recyclage pour emballages polymères, dans lequel le procédé (P) est appliqué à des emballages polymères (E) comprenant du PET, du PE, du PP et de l'aluminium, avec les étapes consécutives :
(i) granulation (1) de l'emballage polymère (E) ;
(ii) immersion (2) de l'emballage polymère (E) dans un bain d'éthylène glycol ;
(iii) dissolution sélective (3) de l'emballage polymère (E) ;
(iv) séparation (4) des polymères, comprenant les sous-étapes de séparation de l'aluminium (4.1) et de séparation de PE/PP (4.2) ;
(v) lavage (5) des polymères après l'étape de séparation (4), en éliminant les résidus de glycol présents à la fois dans le PE et le PP ;
(vi) broyage (6) des polymères avant l'étape de lavage (5) ;
(vii) nettoyage et séchage (7) de l'aluminium après la sous-étape de séparation de l'aluminium (4.1) ;
les étapes de lavage (5) et de broyage (6) sont effectuées dans un équipement industriel capable de couper et de broyer l'emballage (E), ayant des conduits avec de l'eau et un système de centrifugeuse et séchage par soufflage d'air.

2. **Le procédé,** selon la revendication 1, ***caractérisé en* ce *que*** le glycol comprend la structure moléculaire I et le PET comprend la structure moléculaire II : 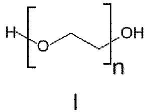 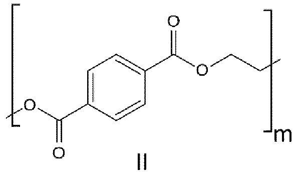 dans lesquelles :
- « n » est égal à des valeurs comprises entre 1 et 10, de préférence entre 1 et 3 ; et
- « m » est égal ou supérieur à 1.

3. **Le procédé,** selon la revendication 1, ***caractérisé en* ce que** l'étape de granulation (1) fragmente l'emballage polymère (E) en petites portions dans un équipement industriel capable de couper et de broyer l'emballage (E), ayant des conduits avec de l'eau et un système de centrifugeuse et séchage par soufflage d'air.

4. **Le procédé,** selon les revendications 1 et 3, ***caractérisé en ce que*** l'étape d'immersion (2) est effectuée après l'étape de granulation (1) dans un réacteur chimique de préférence construit en acier inoxydable et dans une atmosphère contrôlée avec N₂, et le réacteur chimique ayant une entrée supérieure pour la purge d'azote à un débit compris entre cinq et dix L/min ; et un fond conique ou torisphérique.

5. **Le procédé,** selon la revendication 4, ***caractérisé en ce que*** l'étape d'immersion (2) est effectuée dans un bain de glycol pur, dans un état de récipient fermé, et le volume d'emballage (E) doit respecter la plage entre 40 et 60 % de volume de glycol.

6. **Le procédé,** selon la revendication 4, ***caractérisé en ce que*** l'étape d'immersion (2) peut comprendre des sous-étapes de chauffage et d'agitation, dans lequel la sous-étape d'agitation a lieu à travers un système comprenant un moteur et un motoréducteur.

7. **Le procédé,** selon la revendication 6, ***caractérisé en ce que*** la sous-étape de chauffage est effectuée à travers un réchauffeur de fluide thermique contenant un serpentin, élevant la température du glycol à une plage entre 180 °C et 240 °C.

8. **Le procédé,** selon les revendications 1 et 6, ***caractérisé en ce que*** l'étape de dissolution sélective (3) est effectuée après la stabilisation de la température du glycol dans l'étape d'immersion (2) et a une durée d'entre vingt et soixante minutes.

9. **Le procédé,** selon la revendication 8, ***caractérisé en* ce que** l'étape de dissolution sélective (3) est accélérée par des hélices de type Cowles, la circulation et/ou le pompage d'engrenages ou la circulation à travers des écrans métalliques.

10. Le **procédé,** selon la revendication 8, ***caractérisé en* ce *que*** le produit de l'étape de dissolution sélective (3) sera une suspension de PP et de PE ; de l'aluminium précipité sous sa forme métallique ; et d'au moins un polyol à base d'acide téréphtalique comprenant la structure moléculaire III ou la structure moléculaire V : 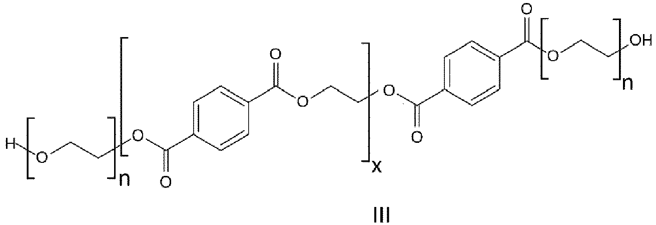 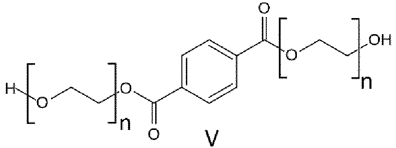 dans lesquelles :
- « n » est égal à des valeurs comprises dans la plage entre 1 et 10, de préférence entre 1 et 3 ;
- « x » est égal ou supérieur à 1 ;

11. Le **procédé,** selon les revendications 1 et 8, ***caractérisé en* ce *que*** l'étape de séparation (4) a lieu après l'étape de dissolution sélective (3) et la fusion complète des films de PP et/ou de PE, et l'étape de séparation (4) comprenant les sous-étapes de séparation d'aluminium (4.1) et de séparation de PE/PP (4.2).

12. Le **procédé,** selon la revendication 11, ***caractérisé en ce que*** la sous-étape de séparation de l'aluminium (4.1) consiste à drainer une petite fraction du bain du fond du réacteur, sous l'effet de la chaleur, puis à faire passer ce bain à travers une ligne de filtration.

13. Le **procédé,** selon la revendication 11, ***caractérisé en ce que*** la sous-étape de séparation de PP/PE (4.2) consiste à drainer le bain et la partie surnageante par pompage, à travers des drains latéraux situés dans le réacteur, à les diriger vers un filtre à tamis métallique fin, à refroidir la partie surnageante et à renvoyer le bain au réacteur par pompage.

14. Le **procédé,** selon les revendications 1 et 4, ***caractérisé en ce que*** le procédé (P) utilise du mono-éthylène glycol, du di-éthylène glycol et du tri-éthylène glycol comme glycol.

15. Le **procédé,** selon les revendications 1 et 14, ***caractérisé en ce que*** l'étape de nettoyage et de séchage de l'aluminium (7) a lieu après la sous-étape de séparation de l'aluminium (4.1), en éliminant les particules de glycol au niveau de l'aluminium par un lavage direct de type rideau d'eau, sur un convoyeur ou une gouttière.
